# EUROPEAN PATENT APPLICATION

(11) **EP 2 305 650 A1**
(43) Date of publication of application: **06.04.2011**
(21) Application number: 09382173.4
(22) Date of filing: 21.09.2009
(51) Int. Cl.: C07D 235/20

(54) **Novel process for the preparation of telmisartan**

(71) Applicant: Chemo Ibérica, S.A., 08028 Barcelona (ES)
(72) Inventor: Castaldi, Graziano, 28072 Briona (NO) (IT); Rasparini, Marcello, 27010 Cura Carpignano (PV) (IT); Carcone, Luca, 03044 Cervaro (FR) (IT); Romanò, Bruno, 23880 Casatenovo (LC) (IT)
(74) Representative: ZBM Patents

(57) **Abstract**

The present invention relates to a novel process for preparing Telmisartan by ester hydrolysis using a silanolate compound.

## Description

### Field of the invention

The present invention relates to a novel process for preparing Telmisartan.

### Background of the invention

Telmisartan and its physiologically acceptable salts have valuable pharmacological properties. Telmisartan is an angiotensin-II-antagonist, which may be used to treat hypertension and cardiac insufficiency, ischaemic peripheral circulatory disorders, myocardial ischaemia (angina). Furthermore, Telmisartan may be used to prevent the progression of cardiac insufficiency after myocardial infarct, to treat diabetic neuropathy, glaucoma, gastrointestinal diseases and bladder diseases. Telmisartan is also suitable for treating pulmonary diseases, e. g. lung oedema and chronic bronchitis, for preventing arterial restenosis after angioplasty, for preventing thickening of blood vessel walls after vascular operations, and for preventing arteriosclerosis and diabetic angiopathy. In view of the effects of angiotensin on the release of acetyl-choline and dopamine in the brain, Telmisartan is also suitable for alleviating central nervous system disorders, e. g. depression, Alzheimer's disease, Parkinson syndrome, bulimia and disorders of cognitive function.

Telmisartan is a compound of formula (I) 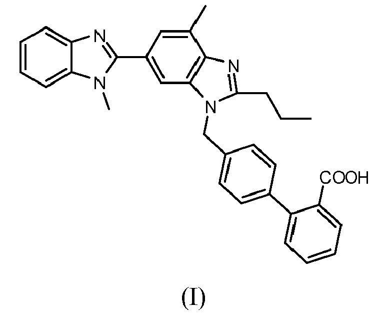 chemically known as 4'-((1,7'-dimethyl-2'-propyl-1*H*,3'*H*-2,5',-bibenzo[*d*]imidazol-3'-yl)methyl)biphenyl-2-carboxylic acid, which is disclosed in EP 502314 B1 and marketed under the trade name Micardis®.

Several methods have been used to prepare Telmisartan.

The process described in EP 502314 B1 comprises the alkylation of 4-methyl-6-(1-methyl-benzimidazol-2-yl)-2-propylbenzimidazole (III) 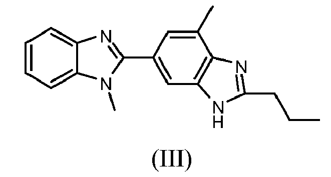 with t-butyl 4'-(bromomethyl)biphenyl-2-carboxylate and subsequently hydrolysis to Telmisartan. t-Butyl 4'-(bromomethyl)biphenyl-2-carboxylate is not commercially available and its synthesis requires a number of steps, among them the protection of the carboxylic function which is finally removed by hydrolysis.

The patent application WO 2006044648 relates to a method for the production of Telmisartan by reacting 4-methyl-6-(1-methyl-benzimidazol-2-yl)-2-propylbenzimidazole (III) with 4'-(bromomethyl)biphenyl-2-carboxylic acid alkyl ester and subsequently hydrolysis.

The patent application WO 2004087676 relates to a method for the production of Telmisartan by reacting 4-methyl-6-(1-methyl-benzimidazol-2-yl)-2-propylbenzimidazole (III) with 4-bromomethyl-2'-cyanobiphenyl and subsequently hydrolysis of the nitrile to the acid function.

The patent application EP 1719766 relates to a method for the production of Telmisartan, by coupling with a Suzuki reaction the *N*-4-bromobenzyl derivative of the compound of formula (III) with 2-carboxylphenyl boronic acid. As described in EP 1878735, 2-carboxyphenyl boronic acid requires a very laborious process to separate it, since it is extremely soluble in water, making the process unattractive for an industrial application. Thus, the active substance prepared by the process known up till now can only be obtained in a satisfactory quality after running through a number of process steps, wherein additional steps of protection and deprotection of the carboxylic function or additional steps to obtain the carboxylic function are often present.

There is therefore the need for an alternative synthesis for the industrial preparation of Telmisartan, which makes use of commercially available reagents and mild conditions.

### Summary of the invention

It has now been found a novel process for the preparation of Telmisartan in a straightforward manner exploiting commercially available starting materials.

The invention relates to a process for preparing Telmisartan, or a polymorph thereof, of formula (I) 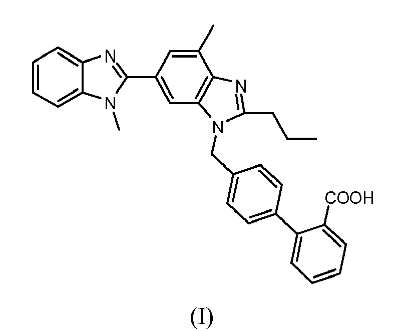 comprising the conversion of a compound of formula (V), 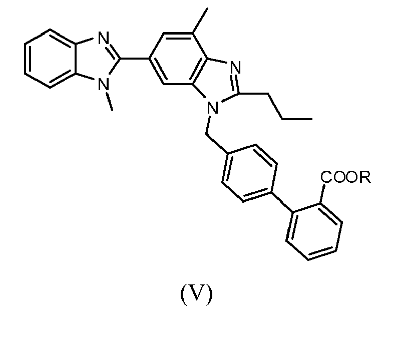 wherein R is a C₁-C₆ alkyl group optionally substituted,
in the presence of a silanolate of formula (R₁)₃OSiM,
wherein R₁ is a C₁-C₆ alkyl group, aryl group optionally substituted with from 1 to 5 substituents selected from the group consisting of C₁-C₈ alkyl, halogen, and aryl, and M is an alkali metal,
and further comprising an acidic hydrolysis.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "C₁-C₆ alkyl" refers to a straight or branched hydrocarbon having from 1 to 6 carbon atoms. Examples of alkyl groups include, without limitation, methyl, ethyl, *n-*propyl, isopropyl, *n*-butyl, *sec*-butyl, isobutyl, *tert*-butyl, *n*-pentyl, *n*-hexyl, and the like. Preferred alkyl groups of the present invention are methyl, ethyl.

The term "aryl" refers to an aromatic carbocyclic group having a single ring (e.g., phenyl), multiple rings (e. g., biphenyl), or multiple condensed rings in which at least one is aromatic (*e*.*g*., 1,2,3,4-tetrahydronaphthyl, 1-naphthyl, 2-naphthyl, anthryl, or phenanthryl). The aryl group may be optionally substituted. A preferable aryl group of the present invention is phenyl.

The term "halogen" refers to chlorine, bromine, fluoride or iodine.

The term "substituted" refers to groups substituted with from 1 to 5 substituents selected from the group consisting of C₁-C₈ alkyl, halogen, aryl, and the like.

The term "C₁-C₈ alkyl" refers to a straight or branched hydrocarbon having from 1 to 8 carbon atoms, hence comprehensive of the aforementioned "C₁-C₆ alkyl" and also comprising *n-, sec-, iso-, tert-,* and *neo*-heptyl and *n-, sec-, iso-, tert-,* and *neo*-octyl, and the like.

The term "leaving group" has the same meaning to the skilled man (Advanced Organic Chemistry: reactions, mechanisms and structure - Third Edition by Jerry March, John Wiley and Sons Ed.; 1985 page 179) and represents a group which is part of and attached to a substrate molecule; in a reaction where the substrate molecule undergoes a displacement reaction (with for example a nucleophile), the leaving group is then displaced. Preferred leaving groups are halogen, such as chloride, bromide, iodide; sulfonic esters, such as mesylate, tosylate, triflate; more preferably bromide or chloride.

The term "solvate" refers to a molecular complex comprising a disclosed or claimed compound and a stoichiometric or non-stoichiometric amount of one or more solvent molecules.

The term "hydrate" refers to a solvate comprising a disclosed or claimed compound and a stoichiometric or non-stoichiometric amount of water.

The term "polymorph" refers to substances that have the same chemical formula, but different crystal structures.

The term "one pot" process refers to a series of consecutive reactions which are carried out without isolating the respective intermediates.

The term "azeotropic distillation" refers to a type of distillation in which a substance is added to the mixture to be separated in order to form an azeotropic mixture with one or more of the constituents of the original mixture. The azeotrope or azeotropes thus formed will have boiling points different from the boiling points of the original mixture.

The term "azeotropic mixture" refers to a liquid mixture of two or more substances which behaves like a single substance in that the vapor produced by partial evaporation of liquid has the same composition as the liquid. The constant boiling mixture exhibits either a maximum or minimum boiling point as compared with that of other mixtures of the same substance.

The term "about" encompasses the range of experimental error that may typically occur in a measurement.

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

In accordance with Scheme 1, the present invention provides a novel process for the preparation of pharmaceutically effective Telmisartan (I). 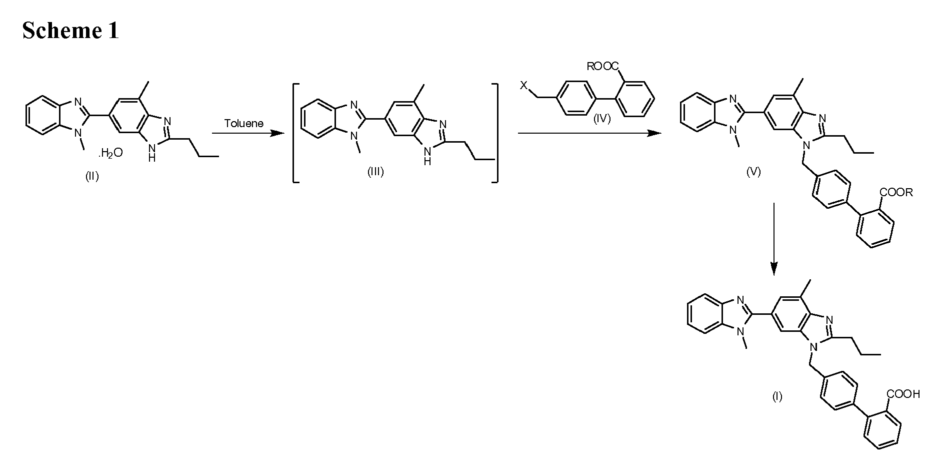 wherein X, R and M are as defined above.

The invention provides the conversion of a compound of formula (V), as defined above, to Telmisartan of formula (I), in the presence of a silanolate of formula (R₁)₃OSiM, wherein R₁ is a C₁-C₆ alkyl group or an aryl group optionally substituted, preferably R₁ is methyl, ethyl, phenyl, more preferably methyl, phenyl; and M is an alkali metal, such as lithium, sodium, potassium, preferably sodium.

The silanolate of formula (R₁)₃OSiM can be generated *in situ* by reacting a silanol of formula (R₁)₃SiOH, wherein R₁ is as defined above, with a basic agent, for example, alkali metal hydrides, such as sodium or potassium hydride; alkali metal hydroxides, such as sodium, potassium hydroxide; in a suitable solvent. Examples of suitable solvents are non polar solvents, for instance aromatic hydrocarbons, such as benzene, toluene; ethers, such as tetrahydrofuran, methyl tetrahydrofuran, dioxane, dimethoxyethane; polar aprotic solvents, such as dimethylacetamide, dimethylformamide, N-methyl pyrrolidone. Preferably the solvent used is toluene. Preferred silanolates of the present invention are sodium trimethylsilanolate, sodium triphenylsilanolate. The silanolate are preferably employed in a molar ratio of 1.0 to 2.0 to the compound of formula (V), preferably in a molar ratio between 1.0 and 1.2.

The reaction may be carried out over a wide range of temperatures to achieve the desired reaction rate. The temperature is generally between room temperature and reflux temperature, preferably between 90 and 120 °C.

In a preferred embodiment, the conversion of a compound of formula (V) is carried out in toluene with sodium trimethylsilanolate or sodium triphenylsilanolate.

To ultimate the conversion, the reaction mixture is then submitted to an acidic hydrolysis at a suitable temperature. A suitable acid is an organic acid, for example, acetic acid, trifluoroacetic acid, formic acid, methanesulfonic, p-toluenesulfonic acid or the like, preferably acetic acid; or an inorganic acid, for example hydrochloric acid, phosphoric acid, nitric acid, sulfuric acid or the like, at a temperature comprised between room temperature and reflux temperature. A preferred range of temperature is between 50 and 90°C.

In a preferred embodiment of the invention, the intermediates of formula (V) are obtained by a process comprising:
a) submitting a compound of formula (II) 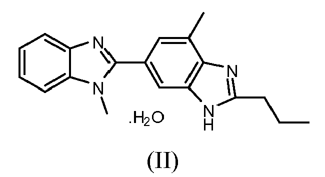 to azeotropic distillation to obtain a compound of formula (III) 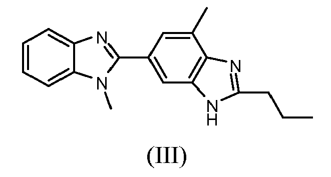
b) reacting a compound of formula (III) 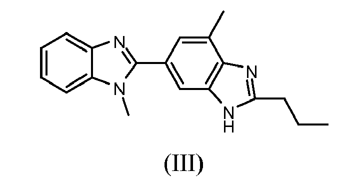 with a compound of formula (IV), 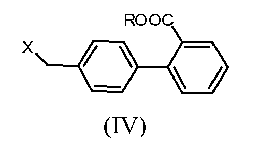 wherein:
   X is a leaving group,
   R is a C₁-C₆ alkyl group optionally substituted ,
   in the presence of a base.

The anhydrous compound of formula (III) can be obtained by submitting the commercially available compound of formula (II) to azeotropic distillation. Any solvent known to those skilled in the art able to form an azeotrope with water may be used for the azeotropic distillation. Preferably, for the azeotropic distillation of the present invention, is used a binary solvent mixture, wherein at least one solvent is water, and the second solvent present in the mixture is any solvent known to those skilled in the art able to form an azeotrope with water, such as an aromatic hydrocarbon, for instance toluene; or an apolar solvent, such as cyclohexane. Preferably the second solvent is toluene.

In a preferred embodiment, stages a) and b) are carried out in a "one pot" reaction to give straight compounds of formula (V). Thus, according to said "one pot" reaction, bis-benzimidazole (III) is not isolated and is directly reacted with a biphenyl derivative of formula (IV), wherein X is a leaving group, preferably bromide or chloride, more preferably bromide, and R is a C₁-C₆ alkyl group optionally substituted, preferably methyl or ethyl, in the presence of a basic agent, in a suitable solvent, for example, polar solvents, such as dimethylformamide, dimethylacetamide, N-methyl pyrrolidone, acetone, methyl isobutyl ketone, methyl ethyl ketone, tetrahydrofuran. Preferred solvents are dimethylacetamide, dimethylformamide.

Preferably, the biphenyl derivative is employed in a molar ratio of 1.0 to 2.0 to the bis-benzimidazole hydrate (II), more preferably in a molar ratio between 1.0 and 1.2.

Examples of suitable basic agents include inorganic bases or organic bases, for instance sodium or potassium carbonate, sodium or potassium hydroxide, potassium, sodium or potassium methoxide, sodium or potassium *tert*-butoxide, potassium tert-pentoxide, potassium n-butoxide, sodium hydride. Preferably the base is sodium *tert-*butoxide. Preferably, the base is employed in a molar ratio of 1.0 to 2.0 to the bis-benzimidazole hydrate (II), preferably in a molar ratio from 1.0 to 1.2.

It is contemplated that the compounds of the present method can be found or isolated in the form of hydrates or solvates, in different polymorphic forms, i.e., different crystalline forms, which are considered to fall within the scope of the present invention.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
DMA (N,N-dimethylacetamide); EtOAc (Ethyl Acetate); *t*BuONa (Sodium *tert*-butoxide) hrs (hours); MeOH (Methanol); TLC (Thin Layer Chromatography).

### Example 1

### Methyl 4'-((1,7'-dimethyl-2'-propyl-1H,3'H-2,5'-bibenzo[d]imidazol-3'-yl)methyl)biphenyl-2-carboxylate (V)

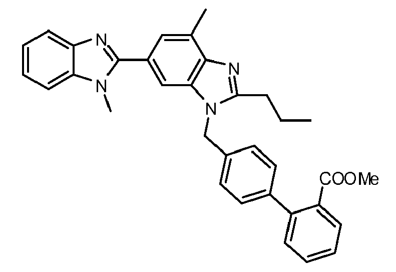

A 1L four-necked glass vessel, fitted with mechanical stirrer, thermometer, dropping funnel, Dean-Stark apparatus, was charged with 1,7'-dimethyl-2'-propyl-1*H*,3*H*-2,5'-bibenzo[*d*]imidazole hydrate (II) (100 g, 310 mmol) and toluene (400 mL), under nitrogen atmosphere. The resulting mixture was heated under stirring to reflux temperature and the water was eliminated by azeotropic distillation, until a semi-solid stirrable residue was obtained. To the residue was added DMA (200 mL) and the mixture was heated to 30-40°C until a complete dissolution of the residue. The resulting solution was then cooled at 0-5°C, tBuONa (31 g, 322 mmol) and methyl 4'-(bromomethyl)biphenyl-2-carboxylate (IV) (97 g, 318 mmol) were added in portions. The suspension was stirred at room temperature for 2 hrs, and monitored by quantitative TLC (elution with 5% MeOH in EtOAc) until complete conversion. Water (500mL) and toluene (300 mL) were then added and the resulting solution was heated to 80°C until a biphasic system was obtained. The phases were separated and the organic phase was washed with water (3 x 50 mL), dried over sodium sulphate and concentrated until a residual volume of 300 mL was reached. The solution was then quickly cooled until crystallization and the so-obtained solid was filtered and washed with toluene (2 x 40 mL). The collected product was dried at 60°C under reduced pressure, affording the title compound (145 g) as a white solid.
Yield: 88.4%

### Example 2

### 4'-((1,7'-dimethyl-2'-propyl-1H,3'H-2,5'-bibenzo[d]imidazol-3'-yl)methyl)biphenyl-2-carboxylic acid (I)

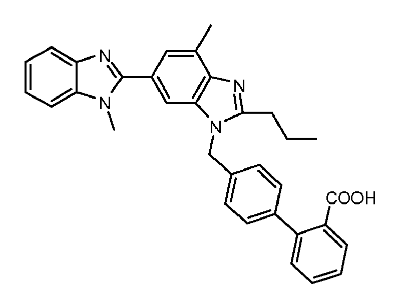

A 2L four-necked glass reactor, fitted with mechanical stirrer, thermometer, dropping funnel, under nitrogen atmosphere, was charged with sodium hydride (60% in mineral oil) (12.5 g, 312 mmol) and toluene (450 mL). The suspension was stirred and trimethylsilanol (31 g, 343 mmol) was added dropwise. After stirring for 15 minutes, methyl 4'-((1,7'-dimethyl-2'-propyl-1*H*, 3'*H*-2,5'-bibenzo[*d*]imidazol-3'-yl)methyl)biphenyl-2-carboxylate (V) (145 g, 274 mmol) was added, the mixture was stirred for 5 hours at about 100°C and monitored by quantitative TLC (elution with 5% MeOH in EtOAc) until complete conversion. The mixture was then cooled at room temperature, water (130 mL) was added, and the mixture was brought at 50°C. The phases were separated and the aqueous phase was stripped under vacuum to remove residual toluene.
350 g of an aqueous solution were obtained and used as such in the next step.

A 1L four-necked glass reactor, fitted with mechanical stirrer, thermometer, dropping funnel, under nitrogen atmosphere, was charged with the aqueous solution in MeOH (600 mL). The mixture was heated under stirring at 40°C until dissolution and charcoal (7 g) was added. The suspension was stirred at 40°C for 30 minutes, filtered through a pad of Celite and the resulting solid was washed with a mixture of MeOH/water 4/1 (100 mL). The filtrate and the washings were combined, the resulting solution was heated to reflux temperature and acetic acid (17.7 g, 295 mmol) was added dropwise over 1 hour. The suspension was then cooled, filtered and the solid was washed with a mixture MeOH/water 4/1 (3 x 50 mL). The collected solid was then dried at 55°C under reduced pressure affording the title compound (130 g) as a white solid.
Yield: 92.2 %

## Claims

1. A process for preparing Telmisartan, or a polymorph thereof, of formula (I), 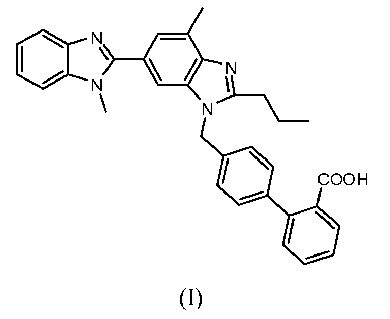 comprising the conversion of a compound of formula (V), 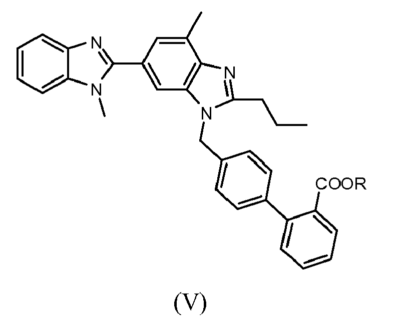 wherein R is a C₁-C₆ alkyl group optionally substituted,
in the presence of a silanolate of formula (R₁)₃OSiM,
wherein R₁ is a C₁-C₆ alkyl group, aryl group optionally substituted with from 1 to 5 substituents selected from the group consisting of C₁-C₈ alkyl, halogen, and aryl, and M is an alkali metal,
and further comprising an acidic hydrolysis.

2. The process according to claim 1, wherein R is methyl, ethyl; R₁ is methyl, ethyl, phenyl; preferably methyl, phenyl; and M is sodium.

3. The process according to claim 1, wherein the silanolate is sodium trimethylsilanolate, sodium triphenylsilanolate.

4. The process according to claim 1, wherein the acid is acetic acid.

5. The process according to claim 1, wherein said compound of formula (V) is prepared by a process comprising:
a) submitting a compound of formula (II) 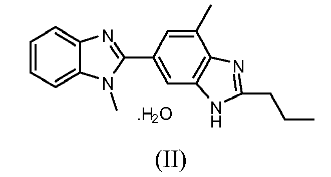 to azeotropic distillation, to obtain a compound of formula (III) 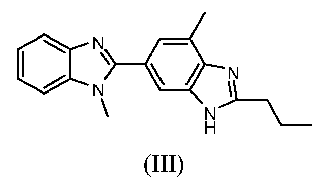
b) reacting a compound of formula (III) with a compound of formula (IV) 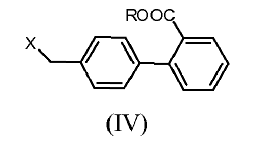 wherein:
X is a leaving group,
R is a C₁-C₆ alkyl group optionally substituted with from 1 to 5 substituents selected from the group consisting of C₁-C₈ alkyl, halogen, aryl,
in the presence of a base.

6. A process according to claim 5, wherein X is bromide, chloride, preferably bromide;
R is methyl, ethyl; and the base is sodium *tert*-butoxide.

7. A process according to claim 5, wherein stages a) and b) are carried out in a "one pot" reaction.
